(19) Europäisches Patentamt
European Patent Office
Office européen des brevets

(11) Veröffentlichungsnummer : **0 434 620 A2**

# (12) EUROPÄISCHE PATENTANMELDUNG

(21) Anmeldenummer : 90810979.6

(22) Anmeldetag : 12.12.90

(51) Int. Cl.[5] : C07D 417/12, A01N 43/78, C07D 277/56

(30) Priorität : 21.12.89 CH 4580/89
26.10.90 CH 3425/90

(43) Veröffentlichungstag der Anmeldung : 26.06.91 Patentblatt 91/26

(84) Benannte Vertragsstaaten : AT BE CH DE DK ES FR GB GR IT LI LU NL SE

(71) Anmelder : CIBA-GEIGY AG
Klybeckstrasse 141
CH-4002 Basel (CH)

(72) Erfinder : Hubele, Adolf, Dr.
Obere Egg 9
CH-4312 Magden (CH)

## (54) Schädlingsbekämpfungsmittel.

(57) Thiazolyl-5-carbonamid-Derivate der Formel I

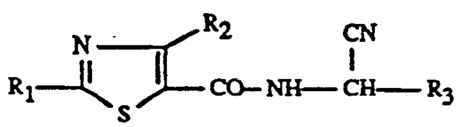

(I)

worin $R_3$ Furanyl oder Thienyl bedeutet und $R_1$ und $R_2$ die hierin genannten Bedeutungen haben, besitzen wertvolle mikrobizide Wirkung. Sie lassen sich in Form von Mitteln zur Bekämpfung bzw. Verhütung von Pflanzenkrankheiten einsetzen.

EP 0 434 620 A2

# SCHÄDLINGSBEKÄMPFUNGSMITTEL

Die vorliegende Erfindung betrifft neue Thiazolyl-5-carbonamid-Derivate der nachstehenden Formel I. Sie betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von Mikroorganismen, vor allem von pflanzenschädigenden Mikroorganismen, vorzugsweise Pilzen.

Die erfindungsgemässen Verbindungen entsprechen der allgemeinen Formel I und schliessen ihre Säureadditionssalze und Metallsalzkomplexe ein.

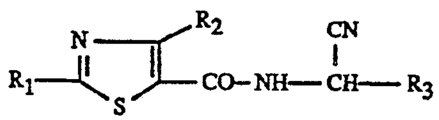

(I)

In dieser Formel bedeuten

$R_3$ 2-Furanyl, 2-Thienyl, 3-Furanyl oder 3-Thienyl ;

$R_1$ und $R_2$ unabhängig voneinander

a) $C_3$-$C_6$Cycloalkyl, das unsubstituiert oder durch Methyl oder Methylthio substituiert ist,

b) die Gruppe $-CH_2$-X-$R_4$, und

c) einer der beiden Substituenten $R_1$ oder $R_2$ auch Wasserstoff oder $C_1$-$C_4$Alkyl, wobei ferner X Sauerstoff oder Schwefel darstellt,

$R_4$ $C_1$-$C_4$Alkyl bedeutet, das unsubstituiert oder durch Halogen oder im Falle $C_2$-$C_4$Alkyl auch durch $C_1$-$C_3$Alkoxy substituiert sein kann, oder worin $R_4$ $C_3$-$C_4$Alkenyl, $C_3$-$C_4$Alkinyl oder eine Phenylgruppe oder Benzylgruppe darstellt, deren aromatischer Ring unsubstituiert ist oder durch Halogen, $C_1$-$C_2$Alkyl, $C_1$-$C_2$Alkoxy, $CF_3$ oder $NO_2$ substituiert sein kann.

Unter Alkyl selbst oder als Bestandteil eines anderen Substituenten, wie Halogenalkyl oder Alkoxy sind je nach Anzahl der benannten Kohlenstoffatome beispielsweise zu verstehen Methyl, Ethyl, Propyl, Butyl, sowie ihre Isomeren Isopropyl, Isobutyl, tert.-Butyl oder sek.-Butyl. Halogen, auch Hal genannt, steht für Fluor, Chlor, Brom oder Jod. Halogenalkyl bezeichnet einfach bis perhalogenierte Reste, wie z.B. $CHCl_2$, $CH_2F$, $CCl_3$, $CH_2Cl$, $CHF_2$, $CF_3$, $CH_2CH_2Br$, $C_2Cl_5$, $CH_2Br$, CHBrCl usw., Cycloalkyl steht je nach Zahl der genannten Kohlenstoffatome z.B. für Cyclopropyl, Cyclobutyl, Cyclopentyl oder Cyclohexyl.

Die Verbindungen der Formel I sind bei Raumtemperatur stabile Oele oder Feststoffe, die sich durch wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen. Die erfindungsgemässen Wirkstoffe der Formel I zeichnen sich bei niedrigen Anwendungskonzentrationen nicht nur durch hervorragende mikrobizide, besonders fungizide Wirkung, sondern auch durch besonders gute Pflanzenverträglichkeit aus.

Nachbarständig zur Nitrilgruppe besitzen Verbindungen der Formel I ein asymmetrisches C-Atom. Sie lassen sich daher auf übliche Art in optische Antipoden spalten. Diese Antipoden besitzen unterschiedliche mikrobizide Wirkung.

Die Erfindung betrifft sowohl die freien Verbindungen der Formel I als auch deren Additionssalze mit anorganischen und organischen Säuren sowie deren Komplexe mit Metallsalzen.

Erfindungsgemässe Salze sind insbesondere Additionssalze mit unbedenklichen anorganischen oder organischen Säuren, beispielsweise Halogenwasserstoffsäuren, z.B. Chlor-, Brom- oder Jodwasserstoffsäure, Schwefelsäure, Phosphorsäure, phosphorige Säure, Salpetersäure, oder organischen Säuren wie Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure, 2-Acetoxybenzoesäure oder 1,2-Naphthalin-disulfonsäure.

Metallsalzkomplexe der Formel I bestehen aus dem zugrundeliegenden organischen Molekül und einem anorganischen oder organischen Metallsalz, z.B. den Halogeniden, Nitraten, Sulfaten, Phosphaten, Acetaten, Trifluoracetaten, Trichloracetaten, Propionaten, Tartraten, Sulfonaten, Salicyclaten, Benzoaten usw. der Elemente der zweiten Hauptgruppe wie Calcium und Magnesium und der dritten und vierten Hauptgruppe wie Aluminium, Zinn oder Blei sowie der ersten bis achten Nebengruppe wie Chrom, Mangan, Eisen, Kobalt, Nickel, Kupfer, Zink usw. Bevorzugt sind die Nebengruppen-Elemente der 4. Periode. Die Metalle können dabei in den

verschiedenen ihnen zukommenden Wertigkeiten vorliegen. Die Metallkomplexe können ein- oder mehrkernig auftreten, d.h. sie können ein oder mehrere organische Molekülanteile als Liganden enthalten.

Eine wichtige Gruppe von Pflanzenfungiziden und Insektiziden sind jene der Formel I, worin einer der beiden Substituenten $R_1$ und $R_2$ $C_3$-$C_6$Cycloalkyl bedeutet, das unsubstituiert oder durch Methyl oder Methylthio substituiert ist, und der andere Substituent

d) Cyclobutyl, Cyclopropyl, Methylcyclopropyl oder Methylthiocyclopropyl,

e) die Gruppe —$CH_2$-X-$R_4$ oder

f) Wasserstoff oder $C_1$-$C_4$Alkyl darstellt, während

$R_3$, X und $R_4$ die vorgenannte Bedeutung haben.

Diese sollen hier und im folgenden Verbindungsgruppe IA genannt werden.

Unter diesen Verbindungen der Gruppe IA sind solche besonders zu nennen, worin einer der beiden Substituenten $R_1$ und $R_2$ $C_3$-$C_6$Cycloalkyl und der andere Cyclobutyl oder Cyclopropyl bedeutet, während $R_3$ die genannte Bedeutung hat (Gruppe IAA), und unter diesen wiederum jene, worin $R_1$ und $R_2$ Cyclopropyl bedeuten.

Wichtige Verbindungen sind auch solche der Gruppe IA, worin

$R_1$ $C_3$-$C_6$Cycloalkyl bedeutet, das unsubstituiert oder durch Methyl oder Methylthio substituiert ist,

$R_2$ Wasserstoff, $C_1$-$C_4$Alkyl oder

die Gruppe —$CH_2$-X-$R_4$ darstellt,

X Sauerstoff oder Schwefel bedeutet, und

$R_4$ $C_1$-$C_4$Alkyl bedeutet, das unsubstituiert oder durch Fluor substituiert ist, oder im Falle $C_2$-$C_4$Alkyl auch durch $C_1$-$C_3$Alkoxy substituiert sein kann ; oder worin $R_4$ Allyl, Propargyl, Phenyl oder Benzyl darstellt, wobei die aromatischen Ringe der letztgenannten Reste unsubstituiert oder durch einen oder zwei der Substituenten Fluor, Chlor, Methyl, Ethyl, Methoxy oder $CF_3$ substituiert sein können, während $R_3$ die vorgenannte Bedeutung hat (Gruppe IB).

Bevorzugt sind solche Verbindungen der Gruppe IB, worin X Sauerstoff bedeutet (Gruppe IBB).

Wichtige Verbindungen innerhalb der Gruppe IBB sind solche, worin

$R_1$ Cyclobutyl, Cyclopropyl, Methylcyclopropyl oder Methylthiocyclopropyl bedeutet,

$R_2$ Wasserstoff, $C_1$-$C_4$Alkyl oder —$CH_2$-O-$R_4$ darstellt, worin

$R_4$ $C_1$-$C_4$Alkyl bedeutet, das unsubstituiert oder durch Fluor, Methoxy oder Ethoxy substituiert ist, oder worin $R_4$ Allyl, Propargyl, Phenyl, Chlorphenyl, Fluorphenyl, Benzyl, Chlorbenzyl oder Fluorbenzyl bedeutet (= Untergruppe Ib).

Innerhalb der Gruppe Ib sind solche Verbindungen bevorzugt, worin

$R_1$ Cyclopropyl bedeutet,

$R_2$ Wasserstoff, $C_1$-$C_4$Alkyl oder —$CH_2$-O-$R_4$ darstellt, worin

$R_4$ Methyl, Ethyl, Fluormethyl, Difluormethyl, Trifluorethyl, Allyl, Propargyl, Phenyl oder Benzyl ist, während

$R_3$ die genannte Bedeutung hat (= Untergruppe Ibb).

Besonders bevorzugte Verbindungen innerhalb der Gruppe Ibb sind diejenigen, worin

$R_2$ Wasserstoff, $C_1$-$C_3$Alkyl, Methoxymethyl oder Ethoxymethyl bedeutet.

Wichtige Verbindungen sind auch solche der Gruppe Ia, worin

$R_1$ Wasserstoff, $C_1$-$C_4$Alkyl oder

die Gruppe —$CH_2$-X-$R_4$ darstellt,

X Sauerstoff oder Schwefel bedeutet, und

$R_4$ $C_1$-$C_4$Alkyl bedeutet, das unsubstituiert oder durch Fluor substituiert ist, oder im Falle $C_2$-$C_4$Alkyl auch durch $C_1$-$C_3$Alkoxy substituiert sein kann ; oder worin $R_4$ Allyl, Propargyl, Phenyl oder Benzyl darstellt, wobei die aromatischen Ringe der letztgenannten Reste unsubstituiert oder durch einen oder zwei der Substituenten Fluor, Chlor, Methyl, Ethyl, Methoxy oder $CF_3$ substituiert sein können, während

$R_2$ $C_3$-$C_6$Cycloalkyl bedeutet, das unsubstituiert oder durch Methyl oder Methylthio substituiert ist, während $R_3$ die genanne Bedeutung hat (= Gruppe IC), insbesondere jene, worin X Sauerstoff bedeutet (= Gruppe ICC).

Innerhalb dieser Gruppe ICC sind solche Verbindungen bevorzugt, worin

$R_1$ Wasserstoff, $C_1$-$C_4$Alkyl oder —$CH_2$-O-$R_4$ bedeutet,

$R_2$ Cyclobutyl, Cyclopropyl, Methylcyclopropyl oder Methylthiocyclopropyl darstellt, und

$R_4$ $C_1$-$C_4$Alkyl bedeutet, das unsubstituiert oder fluorsubstituiert ist oder, im Falle $C_2$-$C_4$Alkyl, auch durch Methoxy oder Ethoxy substituiert sein kann ; oder worin ferner

$R_4$ Allyl, Propargyl, Phenyl, Chlorphenyl, Fluorphenyl, Benzyl, Chlorbenzyl oder Fluorbenzyl ist, während

$R_3$ die genannte Bedeutung hat (= Untergruppe Ic).

Besonders bevorzugt sind jene Verbindungen innerhalb der Untergruppe Ic, worin

$R_2$ Cyclopropyl bedeutet, und

$R_4$ Methyl, Ethyl, Fluormethyl, Difluormethyl, Trifluorethyl, Allyl, Propargyl, Phenyl oder Benzyl ist (= Untergruppe Icc).

Unter diesen sind solche Verbindungen besonders hervorzuheben, bei denen

$R_1$ Wasserstoff, $C_1$-$C_3$Alkyl, Methoxymethyl oder Ethoxymethyl bedeutet.

Wichtige Verbindungen sind auch jene Thiazolyl-5-carbonamid-Derivate der Formel I, worin einer der beiden Substituenten $R_1$ und $R_2$ die Gruppe —$CH_2$-X-$R_4$ und der andere Substituent Wasserstoff, $C_1$-$C_4$Alkyl oder —$CH_2$-X-$R_4$ bedeutet,

X Sauerstoff oder Schwefel darstellt,

$R_3$ die genannte Bedeutung hat, und

$R_4$ $C_1$-$C_4$Alkyl bedeutet, das unsubstituiert oder durch Fluor substituiert ist, oder im Falle $C_2$-$C_4$Alkyl auch durch $C_1$-$C_3$Alkoxy substituiert sein kann ; oder worin $R_4$ Allyl, Propargyl, Phenyl oder Benzyl darstellt, wobei die aromatischen Ringe der letztgenannten Reste unsubstituiert oder durch einen oder zwei der Substituenten Fluor, Chlor, Methyl, Ethyl, Methoxy oder $CF_3$ substituiert sein können (= Untergruppe ID).

Unter diesen Verbindungen der Gruppe ID sind solche hervorzuheben, worin

$R_1$ die Gruppe —$CH_2$-O-$R_4$ bedeutet,

$R_2$ Wasserstoff, $C_1$-$C_4$Alkyl oder —$CH_2$-O-$R_4$ ist,

$R_3$ die genannte Bedeutung hat, und

$R_4$ $C_1$-$C_4$Alkyl bedeutet, das unsubstituiert oder durch Fluor, Methoxy oder Ethoxy substituiert ist, oder worin $R_4$ Allyl, Propargyl, Phenyl, Chlorphenyl, Fluorphenyl, Benzyl, Chlorbenzyl oder Fluorbenzyl bedeutet (Untergruppe IDD).

Bevorzugt sind in der Untergruppe IDD solche Verbindungen der Formel I, worin

$R_4$ Methyl, Ethyl, Fluormethyl, Difluormethyl, Trifluorethyl, Allyl, Propargyl, Phenyl oder Benzyl ist (= Untergruppe Id).

Besonders bevorzugt sind in der letztgenannten Untertruppe Id Verbindungen, worin

$R_2$ Wasserstoff oder $C_1$-$C_3$Alkyl bedeutet.

Besonders bevorzugt sind in der Untergruppe Id auch Verbindungen, worin

$R_1$ und $R_2$ unabhängig voneinander Methoxymethyl oder Ethoxymethyl bedeuten.

Weiterhin sind unter den Verbindungen der Gruppe ID solche Verbindungen hervorzuheben, worin

$R_1$ Wasserstoff, $C_1$-$C_4$Alkyl oder die Gruppe —$CH_2$-O-$R_4$ ist,

$R_2$ —$CH_2$-O-$R_4$ bedeutet,

$R_3$ die genannte Bedeutung hat und

$R_4$ $C_1$-$C_4$Alkyl bedeutet, das unsubstituiert oder durch Fluor, Methoxy oder Ethoxy substituiert ist, oder worin $R_4$ Allyl, Propargyl, Phenyl, Chlorphenyl, Fluorphenyl, Benzyl, Chlorbenzyl oder Fluorbenzyl bedeutet (= Untergruppe IE).

Bevorzugt sind unter den Verbindungen der Gruppe IE diejenigen, worin

$R_4$ Methyl, Ethyl, Fluormethyl, Difluormethyl, Trifluorethyl, Allyl, Propargyl, Phenyl oder Benzyl ist (= Untergruppe IEE).

Besonders bevorzugt unter den Verbindungen der Gruppe IEE sind diejenigen, bei denen

$R_1$ Wasserstoff oder $C_1$-$C_3$Alkyl bedeutet.

Eine wichtige Gruppe von Pflanzenfungiziden sind weiterhin solche der Formel I worin entweder

a) $R_1$ $CH_3$, $C_2H_5$, n-$C_3H_7$, iso$C_3H_7$, $CH_2$-O-$CH_3$, $CH_2$-O-$C_2H_5$ oder $CH_2$-O-$CHF_2$ oder Cyclopropyl; und

$R_2$ Cyclopropyl bedeuten, oder worin

b) $R_1$ $CH_2$-O-$CH_3$, $CH_2$-O-$C_2H_5$, $CH_2$-O-$CHF_2$ oder Cyclopropyl und

$R_2$ $C_1$-$C_3$-Alkyl darstellen, und

$R_3$ die genannte Bedeutung hat (Untergruppe Alpha).

Unter diesen Verbindungen der Gruppe Alpha ist als eine der bevorzugten Untergruppen jene zu nennen, worin

$R_1$ $CH_3$, $C_2H_5$, n-$C_3H_7$ oder-iso-$C_3H_7$, und

$R_2$ Cyclopropyl bedeuten (Untergruppe Beta).

Weiterhin ist unter den Verbindungen der Gruppe Alpha als weitere bevorzugte Untergruppe jene zu nennen, worin

$R_1$ = $CH_2$-O-$CH_3$, $CH_2$-O-$C_2H_5$ oder Cyclopropyl und

$R_2$ = $CH_3$, $C_2H_5$, n-$C_3H_7$, iso-$C_3H_7$ oder Cyclopropyl bedeuten (Untergruppe Gamma).

Verbindungen der Formel I werden erhalten durch Reaktion der Thiazolyl-5-carbonsäure der Formel V

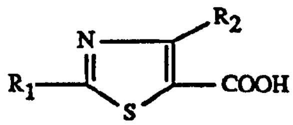

(V)

oder ihrem Säurehalogenid, Ester oder Säureanhydrid mit einem Aminoacetonitril der Formel II

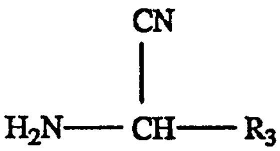

. (II)

in Gegenwart oder Abwesenheit eines Kondensationsmittel bzw. eines Säureakzeptors, wobei die Substituenten $R_1$, $R_2$ und $R_3$ die für die Formel I gegebene Bedeutung haben.

Es ist zweckmässig, die Aminkomponente II im leichten Ueberschuss gegenüber der Komponente V einzusetzen (z.B. bis zu 2 Moläquivalenten).

Als Säurehalogenid ist vor allem das Chlorid, Bromid oder Jodid zu verstehen, als Säureester sind die leicht reaktionsfähigen Derivate der $C_1$-$C_8$Alkohole zu verstehen, z.B.

Methyl-, Ethyl-, Propyl-, Isopropyl-, Butyl-, tert.Butyl-, Amylester und andere. Die Reaktion von V mit II erfolgt in Abwesenheit oder bevorzugt in Gegenwart eines Kondensationsmittels bzw. eines Säureakzeptors (Protonenakzeptors).

Ein Säurehalogenid der Formel V lässt sich auch in situ aus der Thiazolyl-5-carbonsäure durch Reaktion dieser mit dem Reaktanden II in Gegenwart von Thionylhalogenid, z.B. $SOCl_2$, und Imidazol gewinnen. Diese Verfahrensvariante ist im vorgenannten Prozess mit eingeschlossen.

Der Temperaturbereich der Reaktion liegt bei -20°C bis 150°C, bevorzugt bei -5°C bis +80°C.

Als Protonenakzeptoren dienen z.B. anorganische oder organische Basen, wie beispielsweise Alkali- oder Erdalkaliverbindungen, z.B. die Hydroxide, Oxide oder Karbonate von Lithium, Natrium, Kalium, Magnesium, Calcium, Strontium und Barium oder auch Hydride wie z.B. Natriumhydrid. Als organische Basen seien beispielsweise tertiäre Amine wie Triethylamin, Triethylendiamin, Pyridin, 4-Dimethylaminopyridin genannt.

Obgleich nicht zwingend notwendig, lassen sich zur Reaktion Lösungs- bzw. Verdünnungsmittel vorteilhaft verwenden. Es können z.B. genannt werden : Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, 1,2-Dichlorethan, 1,1-Dichlorethan, 1,2-cis-Dichlorethylen, Chlorbenzol, Fluorbenzol, Brombenzol, Dichlorbenzol, Dibrombenzol, Chlortoluol, Trichlortoluol ; Ether, wie Ethylpropylether, Methyl-tert.-butylether, n-Butylethylether, Di-n-butylether, Di-isobutylether, Diisoamylether, Diisopropylether, Anisol, Cyclohexylmethylether, Diethylether, Ethylenglykoldimethylether, Tetrahydrofuran, Dioxan, Thioanisol, Dichlordiethylether, Nitrokohlenwasserstoffe wie Nitromethan, Nitroethan, Nitrobenzol, Chlornitrobenzol, o-Nitrotoluol ; Nitrile wie Acetonitril, Butyronitril, Isobutyronitril, Benzonitril, m-Chlorbenzonitril ; aliphatische oder cycloaliphatische Kohlenwasserstoffe, wie Heptan, Hexan, Octan, Nonan, Cymol, Benzinfraktionen innerhalb eines Siedepunktintervalles von 70°C bis 190°C, Cyclohexan, Methylcyclohexan, Dekalin, Petrolether, Ligroin, Trimethylpentan, wie 2,3,3-Trimethylpentan ; Ester wie Ethylacetat, Acetessigester, Isobutylacetat ; Amide, z.B. Formamid, Methylformamid, Dimethylformamid ; Ketone, wie Aceton, Methylethylketon ; gegebenenfalls auch Wasser. Auch Gemische der genannten lösungs- und Verdünnungsmittel kommen in Betracht.

Die Ausgangsprodukte der Formel II lassen sich nach der Strecker-Synthese durch Reaktion des Aldehyds III mit Blausäure bzw. Alkalicyanid (z.B. NaCN) in Gegenwart von Ammoniak oder einem Ammoniumsalz gewinnen :

$$\underset{\text{(III)}}{R_3\text{ - CHO}} + \text{HCN/Alkalicyanid} \xrightarrow{NH_3/NH_4Cl} \text{II}$$

Man verwendet zweckmässig ein binäres lösungsmittelsystem bestehend einerseits aus einem Ether (Diethylether, Dioxan, THF etc.) oder einem aromatischen Kohlenwasserstoff (Benzol, Toluol, Xylol) und andererseits aus Wasser und arbeitet bei 0° bis 100°C.

Die Ausgangsprodukte der Formel V werden erhalten, indem man ein Thioamid der Formel VI mit einem α-Halogen-β-ketoester der Formel VII zum Thiazolyl-5-carbonsäureester Va umsetzt:

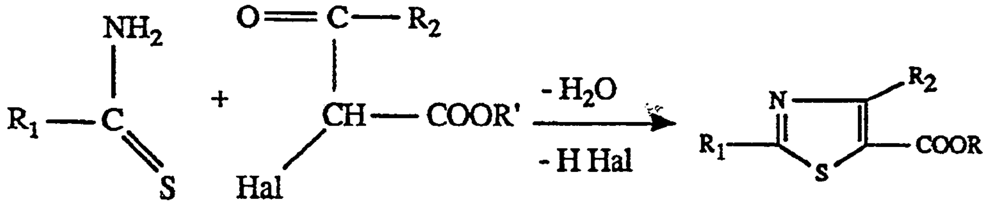


VI VII Va

wobei R' einen $C_1$-$C_6$ Kohlenwasserstoff bedeutet, und anschliessend, sofern gewünscht, durch Verseifung in die Carbonsäure V überführt. Die Reaktion kann in einem protischen lösungsmittel wie Alkohol oder in einem aprotischen lösungsmittel wie Benzol, Toluol, Cyclohexan usw. durchgeführt werden. In einigen Fällen ist es angebracht, einen Protonenakzeptor wie Na-Acetat/Essigsäure oder eint tert.Base (z.B. Pyridin, Triethylamin usw.) zuzusetzen. Die Reaktion wird zunächst bei Temperaturen zwischen -60°C und +40°C, bevorzugt bei -20°C bis +20°C, durchgeführt. Anschliessend wird auf +30°C bis +140°C erwärmt, bevorzugt auf +50°C bis +110°C. Wird z.B. in Benzol, Toluol oder Cyclohexan gearbeitet, kann das sich bildende Wasser mit einem Wasserabscheider entfernt werden.

Nach einer anderen Verfahrensvariante kann das Zwischenprodukt der Formel V auch so gewonnen werden, dass man das Thioamid der Formel VI zuerst mit einem Säurehalogenid der Formel VIII zu einem N-Acylthioamid der Formel IX acyliert:

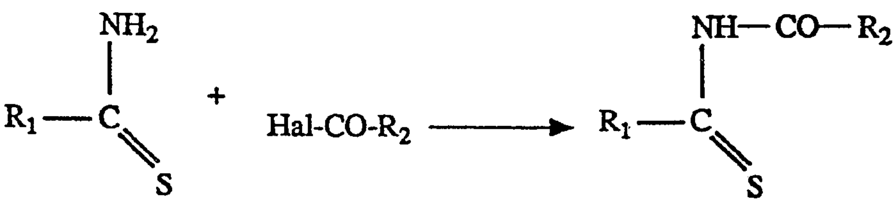

VI VIII IX

Dabei arbeitet man zweckmässig in einem inerten lösungsmittel wie Acetonitril oder Tetrahydrofuran. Als Protonenakzeptor dient bevorzugt eine tert.Base wie Pyridin, Triethylamin, 4-Dimethylaminopyridin u.a. Die Reaktion sollte im Temperaturbereich von -40°C bis +80°C, bevorzugt -20°C bis +20°C, ablaufen. Danach wird IX mit einem Halogenester X zum Thiazolyl-5-carbonsäureester Va kondensiert und dann, sofern gewünscht, in üblicher Weise in V überführt:

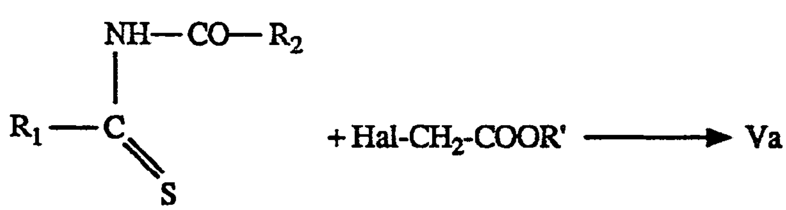

IX X

Bei der Cyclisierung wird das N-Acylthioamid IX in einem protischen Lösungsmittel (wie z.B. einem niederen Alkohol) oder aprotischen lösungsmittel mit einem Alkoholat (z.B. $CH_3$-O-Na) oder mit NaH bei Temperaturen von -40°C bis +20°C, bevorzugt -30°C bis 0°C, behandelt und anschliessend mit dem Halogenester X

bei -20°C bis +30°C, bevorzugt bei -10°C bis +10°C, versetzt. Danach wird, zur Abscheidung des Reaktionswassers, mehrere Stunden auf +40 bis +120°C, bevorzugt +60°C bis +100°C, erhitzt.

In den vorgenannten Formeln Va bis X haben $R_1$ und $R_2$ die für Formel I gegebene Bedeutung.

Die beschriebenen Herstellungsverfahren einschliesslich aller Teilschritte sind ein Bestandteil der vorliegenden Erfindung.

Die neuen Zwischenprodukte der Formel V und ihre Ester mit $C_1$-$C_6$Alkoholen sind ein weiterer Bestandteil vorliegender Erfindung, bevorzugt solche, worin $R_1$ und $R_2$ die für die Untergruppe Alpha gegebenen Bedeutungen haben.

Ueberraschenderweise wurde nun gefunden, dass Verbindungen der Formel I ein für praktische Bedürfnisse sehr günstiges biozides Spektrum zur Bekämpfung von phytopathogenen Mikroorganismen, insbesondere Fungi, aufweisen. Sie besitzen sehr vorteilhafte kurative, präventive und insbesondere systemische Eigenschaften und werden zum Schutz von zahlreichen Kulturpflanzen eingesetzt. Mit den Wirkstoffen der Formel I können an Pflanzen oder Pflanzenteilen (Früchte, Blüten, laubwerk, Stengel, Knollen, Wurzeln) von unterschiedlichen Nutzkulturen die auftretenden Schädlinge eingedämmt oder vernichtet werden, wobei auch später zuwachsende Pflanzenteile z.B. vor phytopathogenen Mikroorganismen verschont bleiben.

Verbindungen der Formel I sind z.B. gegen die den folgenden Klassen angehörenden phytopathogenen Pilze wirksam : Fungi imperfecti (insbesondere Botrytis, ferner Pyricularia, Helminthosporium, Fusarium, Septoria, Cercospora und Alternaria) ; Basidiomyceten (z.B. Rhizoctonia, Hemileia, Puccinia). Darüber hinaus wirken sie gegen die Klasse der Ascomyceten (z.B. Venturia und Erysiphe, Podosphaera, Monilinia, Uncinula), vor allem aber gegen die der Oomyceten (z.B. Phytophthora, Peronospora, Bremia, Pythium, Plasmopara). Die Verbindungen der Formel I können ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft auch die Mittel, die als Wirkstoffkomponente Verbindungen der Formel I enthalten, insbesondere pflanzenschützende Mittel, sowie deren Verwendung auf dem Agrarsektor oder verwandten Gebieten.

Darüber hinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der neuen Verbindungen der Formel I bzw. der neuen Mittel auszeichnet.

Als Zielkulturen für den hierin offenbarten pflanzenschützenden Einsatz gelten im Rahmen dieser Erfindung beispielsweise folgende Pflanzenarten : Getreide (Weizen, Gerste, Roggen, Hafer, Reis, Mais, Sorghum und verwandte Species) ; Rüben (Zucker- und Futterrüben) ; Kern—, Stein und Beerenobst (Aepfel, Birnen, Pflaumen, Pfirsiche, Mandeln, Kirschen, Erdbeeren, Stachelbeeren, Himbeeren und Brombeeren) ; Hülsenfrüchte (Bohnen, Linsen, Erbsen, Soja) ; Oelkulturen (Raps, Senf, Mohn, Oliven, Sonnenblumen, Kokos, Rizinus, Kakao, Erdnüsse) ; Gurkengewächse (Kürbis, Gurken, Melonen) ; Fasergewächse (Baumwolle, Flachs, Hanf, Jute) ; Citrusfrüchte (Orangen, Zitronen, Pampelmusen, Mandarinen) ; Gemüsesorten (Spinat, Kopfsalat, Spargel, Kohlarten, Möhren, Zwiebeln, Tomaten, Kartoffeln, Paprika) ; Lorbeergewächse (Avocado, Cinnamonium, Kampfer) oder Pflanzen wie Tabak, Nüsse, Kaffee, Zuckerrohr, Tee, Pfeffer, Weinreben, Hopfen, Eierfrüchte, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelemente-Vermittler oder andere das Pflanzenwachstum beeinflussende Präparate sein. Es können dabei auch selektive Herbizide sowie Insektizide, Fungizide, Bakterizide, Nematizide, Molluskizide oder Gemische mehrerer dieser Präparate zusammen mit gegebenenfalls weiteren in der Formulierungstechnik üblichen Trägerstoffen, Tensiden oder anderen applikationsfördernden Zusätzen Verwendung finden.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffs der Formel I bzw. eines agrochemischen Mittels, das mindestens einen dieser Wirkstoffe enthält, ist das Aufbringen auf das Blattwerk (Blattapplikation). Applikationsfrequenz und Aufwandmenge richten sich dabei nach dem Befallsdruck des betreffenden Erregers. Die Wirkstoffe der Formel I können aber auch über den Erdboden durch das Wurzelwerk in die Pflanze gelangen (systemische Wirkung), indem man den Standort der Pflanze mit einer flüssigen Zübereitung tränkt oder die Substanzen in fester Form in den Boden einbringt, z.B. in Form von Granulat (Bodenapplikation). Bei Wasserreiskulturen kann man solche Granulate dem überfluteten Reis-Feld zudosieren. Die Verbindungen der Formel I können aber auch auf Samenkörner aufgebracht werden (Coating), indem man die Körner entweder in

einer flüssigen Zubereitung des Wirkstoffs tränkt oder sie mit einer festen Zubereitung beschichtet. Grundsätzlich kann jede Art von Vermehrungsgut einer Pflanze mit Verbindungen der Formel I geschützt werden, z.B. das Saatgut.

Die Verbindungen der Formel I werden dabei in unveränderter Form oder vorzugsweise zusammen mit den in der Formulierungstechnik üblichen Hilfsmitteln eingesetzt. Dafür werden sie zweckmässigerweise z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha, bevorzugt 25 g bis 2 kg AS/ha, insbesondere bei 30 g bis 300 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel kommen in Frage : Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctyl- phthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorilonit oder Attapulgit.

Besonders vorteilhafte applikationsfördernde Zuschlagstoffe, die zu einer starken Reduktion der Aufwandmenge führen können, sind ferner natürliche (tierische oder pflanzliche) oder synthetische Phospholipide aus der Reihe der kephaline und Lecithine, die man beispielsweise aus Sojabohnen gewinnen kann.

Als oberflächenaktive Verbindungen kommen je nach Art des zu formulierenden Wirkstoffs der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-Methyllaurinsalze zu erwähnen.

Als nichtionische Tenside kommen Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Als Beispiele nichtionischer Tenside seien Nonylphenolpolyethoxyethanole, Ricinusölpolyglykolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen.

Weitere in der Formulierungstechnik gebräuchlichen Tenside sind dem Fachmann bekannt oder können der einschlägigen Fachliteratur entnommen werden.

Die in der Formulierungstechnik gebräuchlichen anionischen, nichtionischen oder kationischen Tenside sind dem Fachmann bekannt oder können der einschlägigen Fachliteratur entnommen werden :

– "Mc Cutcheon's Detergents and Emulsifiers Annual", Mc Publishing Corp., Glen Rock, New Jersey, 1988.
– M. and J. Ash, "Encyclopedia of Surfactants", Vol. I-III, Chemical Publishing Co., New York, 1980-1981.
– Dr. Helmut Stache "Tensid-Taschenbuch", Carl Hanser Verlag, München/Wien 1981.

Die agrochemischen Zubereitungen enthalten in der Regel 0,1 bis 99%, insbesondere 0,1 bis 95%, Wirk-

stoff der Formel I, 99,9 bis 1%, insbesondere 99,9 bis 5%, eines festen oder flüssigen Zusatzstoffes und 0 bis 25%, insbesondere 0,1 bis 25%, einesTensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken.

Herstellungsbeispiel 1

a) Herstellung von 2-Methoxymethyl-4-methylthiazol-5-carbonsäureethylester

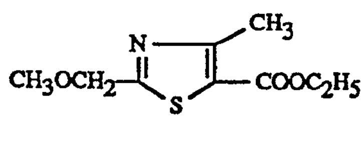

31,3 g 2-Chloracetessigsäureethylester werden in 400 ml Benzol bei Raumtemperatur unter kräftigem Rühren mit 20 g Methoxythioacetamid versetzt und unter Verwendung eines Wasserabscheiders anschliessend $4\frac{1}{2}$ Stunden unter Rückfluss erhitzt. Nach dem Abkühlen auf Raumtemperatur wird die benzolische Reaktionslösung mit 500 ml Ethylacetat verdünnt, zweimal mit je 80 ml 10%iger Sodalösung und zweimal mit je 50 ml Wasser gewaschen, über Natriumsulfat getrocknet und filtriert. Das Lösungsmittelgemisch wird verdampft. Das zurückbleibende gelbe Oel wird durch Anreiben mit Diisopropylether zur Kristallisation gebracht. Die beigefarbenen Kristalle schmelzen bei 36°-37°C.

b) Herstellung von 2-Methoxymethyl-4-methylthiazol-5-carbonsäure

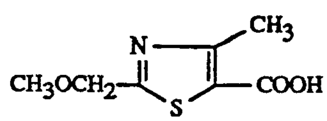

22,1 g 2-Methoxymethyl-4-methylthiazol-5-carbonsäureethylester werden bei Raumtemperatur mit 6,7 g 85%igem Kaliumhydroxyd in 100 ml Ethanol versetzt und anschliessend 18 Stunden unter Rückfluss erhitzt. Nach dem Abkühlen wird das Ethanol abgedampft und der Rückstand in 200 ml Wasser gelöst. Nach dem Verrühren mit Aktivkohle wird das Gemisch über Hyflo filtriert und das Filtrat mit konz. Chlorwasserstoffsäure unter kräftigem Rühren angesäuert. Die ausgefallenen gelblichen Kristalle werden abfiltriert, mit Wasser nachgewaschen und getrocknet. Nach dem Umkristallisieren aus Tetrahydrofuran/Petrolether (30-45°C) (= 10 : 1) schmelzen die gelblichen Kristalle bei 168-169,5°C.

c) Herstellung von 2-(2-Methoxymethyl-3-methylthiazolyl-5-carbonylamino)-2-(3-thienyl)-acetonitril

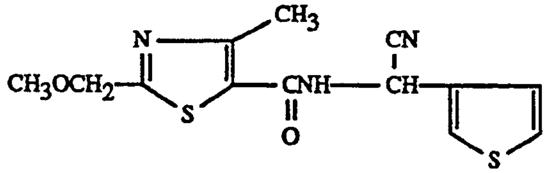

Verb. Nr. 68

Zu 3,5 g Methoxymethyl-4-methylthiazol-5-carbonsäure in 40 ml Pyridin werden unter Rühren bei 0°C innerhalb $\frac{1}{4}$ h 2,38 g Thionylchlorid zugetropft. Nach einstündigem Rühren bei 0°C werden 3,5 g 2-(3-Thienyl)-aminoacetonitrilhydrochlorid zugegeben und 18 Stunden unter Durchleiten von Stickstoff gerührt. Nach der Zugabe von 200 ml Eiswasser und 100 ml Ethylacetat unter Rühren wird mit 50 ml konz. Salzsäure angesäuert, die organische Phase abgetrennt und nochmals mit 70 ml Ethylacetat extrahiert. Die vereinigten Extrakte werden zweimal mit je 100 ml 1 normaler Salzsäure und einmal mit 100 ml gesättigter Natriumbicarbonatlösung gewaschen, über Natriumsulfat getrocknet, filtriert und das Lösungsmittel verdampft. Das zurückbleibende gelbliche Oel wird durch Anreiben mit Diisopropylether zur Kristallisation gebracht. Die blassgelben Kristalle schmelzen bei 111-113°C.

Herstellungsbeispiel 2

Herstellung von 2-(2-Methyl-4-cyclopropylthiazol-5-carbonylamino)-2-(3-thienyl)-acetonitril

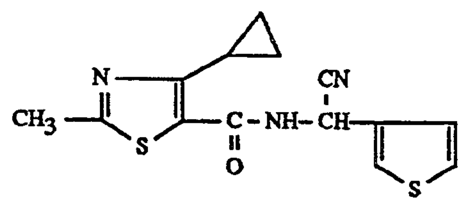

Verb. Nr. 2

8,75 g 2-(3-Thienyl)-aminoacetonitrilhydrochlorid werden in 200 ml Ethylacetat suspendiert und nach dem Abkühlen auf +5°C unter Rühren innerhalb von 5 Minuten mit 12 g Triethylamin versetzt. Unter kräftigem Rühren werden innerhalb 1 Stunde 10 g 2-Methyl-4-cyclopropylthiazol-5-carbonsäurechlorid in 100 ml Ethylacetat bei -5°C zugetropft und anschliessend 2 Stunden bei Raumtemperatur nachgerührt, mit 100 ml Wasser versetzt und die wässrige Phase abgetrennt. die Ethylacetlösung wird noch zweimal mit je 50 ml Wasser gewaschen, über Natriumsulfat getrocknet, filtriert und das Lösungsmittel verdampft. Das zurückbleibende Oel wird säulenchromatographisch über Kieselgel (Tetrahydrofuran : Hexan = 1 : 1) gereinigt. Nach dem Abdampfen des Laufmittelgemisches wird das zunächst bräunliche Oel durch Anreiben mit Petrolether (50-70°C) zur Kristallisation gebracht und aus Hexan/Dichlormethan umkristallisiert. Die beigefarbenen Kristalle schmelzen bei 108-110°C.

In analoger Weise lassen sich folgende Verbindungen herstellen :

Tabelle 1: Verbindungen der Formel

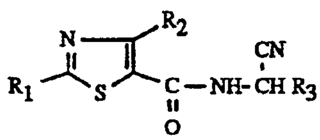

$Q_1$ = 2-Furanyl
$Q_2$ = 3-Furanyl
$Q_3$ = 2-Thienyl
$Q_4$ = 3-Thienyl
CP.= Cyclopropyl

| Verb. Nr. | $R_1$ | $R_2$ | $R_3$ | Physikal. Konstante |
|---|---|---|---|---|
| 1 | H | CP. | $Q_1$ | |
| 2 | $-CH_3$ | CP. | $Q_4$ | Smp. 108-110°C |
| 3 | $-C_2H_5$ | CP. | $Q_3$ | Smp. 116-117°C |
| 4 | H | $-CH_2OCH_3$ | $Q_4$ | |
| 5 | CP. | CP. | $Q_4$ | Smp. 93-95°C |
| 6 | H | CP. | $Q_4$ | |
| 7 | CP. | $-CH_2OCH_3$ | $Q_1$ | |
| 8 | $-CH_3$ | CP. | $Q_1$ | Smp. 67-68°C |
| 9 | $-CH_2OCH_3$ | CP. | $Q_4$ | Smp. 135-136,5°C |
| 10 | $-C_2H_5$ | $-CH_2OCH_3$ | $Q_4$ | |
| 11 | CP. | H | $Q_4$ | |
| 12 | $-CH_3$ | CP. | $Q_2$ | Smp. 91-93°C |
| 13 | 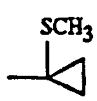 | H | $Q_4$ | |
| 14 | $-CH_2OCH_3$ | H | $Q_1$ | |
| 15 | CP. | $-CH_3$ | $Q_1$ | |
| 16 | CP. | $-CH_2OCH_3$ | $Q_4$ | |
| 17 | $-CH_2OCH_3$ | $-CH_2OCH_3$ | $Q_4$ | |
| 18 | $-CH_3$ | CP. | $Q_3$ | Smp. 135-136°C |

| Verb. Nr. | $R_1$ | $R_2$ | $R_3$ | Physikal. Konstante |
|---|---|---|---|---|
| 19 | $-C_2H_5$ | $-CH_2OC_2H_5$ | $Q_1$ | |
| 20 | Cyclobutyl | $-CH_3$ | $Q_4$ | |
| 21 | $-CH_2OCH_3$ | H | $Q_4$ | |
| 22 | $-CH_3$ | Cyclobutyl | $Q_4$ | |
| 23 | $-CH_2OC_2H_5$ | CP. | $Q_4$ | |
| 24 | CP. | $-CH_3$ | $Q_4$ | Smp. 132-134°C |
| 25 | $-C_2H_5$ | $-CH_2OC_2H_5$ | $Q_4$ | |
| 26 | CP. | $-CH_2OCHF_2$ | $Q_3$ | |
| 27 | $-CH_3$ | $-CH_2OCH_3$ | $Q_1$ | |
| 28 | $-CH_2OCH_3$ | $-CH_2OCHF_2$ | $Q_4$ | |
| 29 | Cyclohexyl | $-CH_3$ | $Q_4$ | |
| 30 | $-CH_2OC_2H_5$ | $-CH_2OCH_3$ | $Q_1$ | |
| 31 | CP. | $-CH_3$ | $Q_2$ | |
| 32 | $-CH_3$ | $-CH_2OCH_3$ | $Q_3$ | Smp. 77-81°C |
| 33 | CP. | $-CH_2OC_2H_5$ | $Q_1$ | |
| 34 | $-C_3H_7$-n | CP. | $Q_4$ | Smp. 114-115°C |
| 35 | $-CH_2OCH_3$ | $-CH_3$ | $Q_1$ | Smp. 80-81°C |
| 36 | $-CH_2OC_2H_5$ | H | $Q_4$ | |
| 37 | $-CH_2OCH_2CH_2OCH_3$ | $-CH_3$ | $Q_4$ | |
| 38 | $-CH_2SCH_3$ | $-CH_3$ | $Q_4$ | |
| 39 | CP. | $-CH_3$ | $Q_3$ | Smp. 127-130°C |
| 40 | $-CH_3$ | $-CH_2OCH_3$ | $Q_2$ | |
| 41 | $-CH_2OC_2H_5$ | $-CH_2OCH_3$ | $Q_4$ | |
| 42 | CP. | $-CH_2OC_2H_5$ | $Q_4$ | |
| 43 | $-CH_2OCHF_2$ | H | $Q_4$ | |

| Verb. Nr. | $R_1$ | $R_2$ | $R_3$ | Physikal. Konstante |
|---|---|---|---|---|
| 44 | $-C_3H_7$-n | $-CH_2OCH_3$ | $Q_4$ | |
| 45 | 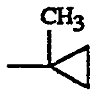 | $-CH_2OCH_3$ | $Q_4$ | |
| 46 | 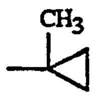 | $-CH_3$ | $Q_4$ | |
| 47 | $-CH_2OCH_2CH_2OCH_3$ | CP. | $Q_4$ | |
| 48 | $-CH_2OCH_3$ | $-CH_3$ | $Q_2$ | Smp. 79-80°C |
| 49 | $-CH_3$ | $-CH_2OCH_3$ | $Q_4$ | |
| 50 | $-CH_2OCH_2CH=CH_2$ | $-CH_3$ | $Q_4$ | |
| 51 | 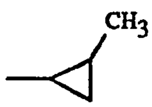 | $-CH_2OCH_3$ | $Q_4$ | |
| 52 | $-CH_2OCH_2CF_3$ | H | $Q_4$ | |
| 53 | $-CH_3$ | $-CH_2OC_2H_5$ | $Q_1$ | |
| 54 | $-CH_2OCH_3$ | $-CH_3$ | $Q_3$ | Smp. 96-98°C |
| 55 | $-C_3H_7$-iso | CP. | $Q_4$ | Smp. 132-133°C |
| 56 | 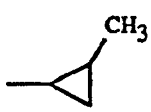 | $-CH_3$ | $Q_4$ | |
| 57 | 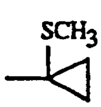 | CP. | $Q_4$ | |
| 58 | $-CH_2OCH_2CH_2OCH_3$ | $-CH_2OCH_3$ | $Q_4$ | |
| 59 | $-CH_3$ | $-CH_2OC_2H_5$ | $Q_4$ | |
| 60 | $-CH_2O-CH_2-C\equiv CH$ | $-CH_3$ | $Q_4$ | |

| Verb. Nr. | $R_1$ | $R_2$ | $R_3$ | Physikal. Konstante |
|---|---|---|---|---|
| 61 | 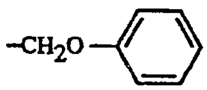 | $-CH_3$ | $Q_4$ | |
| 62 | $-CH_2OCH_2CF_3$ | $-CH_3$ | $Q_4$ | |
| 63 | $-CH_2OCHF_2$ | CP. | $Q_4$ | |
| 64 | 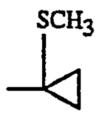 | $-CH_2OCH_3$ | $Q_1$ | |
| 65 | CP. | $-C_2H_5$ | $Q_1$ | |
| 66 | $-CH_3$ | $-CH_2OC_3H_7$-iso | $Q_4$ | |
| 67 | 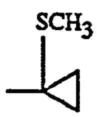 | $-CH_3$ | $Q_1$ | |
| 68 | $-CH_2OCH_3$ | $-CH_3$ | $Q_4$ | Smp. 111-113°C |
| 69 | 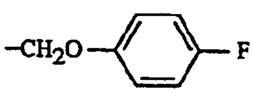 | $-CH_3$ | $Q_4$ | |
| 70 | $-CH_2OC_2H_5$ | $-CH_3$ | $Q_1$ | |
| 71 | 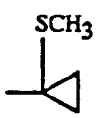 | $-CH_2OCH_3$ | $Q_4$ | |
| 72 | $-CH_2OCHF_2$ | $-CH_2OCH_3$ | $Q_1$ | |
| 73 | $-CH_3$ | $-CH_2OCHF_2$ | $Q_1$ | |
| 74 | 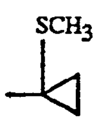 | $-CH_3$ | $Q_3$ | |
| 75 | $-C_3H_7$-iso | $-CH_2OC_2H_5$ | $Q_4$ | |

| Verb. Nr. | $R_1$ | $R_2$ | $R_3$ | Physikal. Konstante |
|---|---|---|---|---|
| 76 | $-CH_2OCH_2-$ 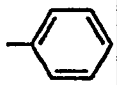 | $-CH_3$ | $Q_4$ | |
| 77 | CP. | $-C_2H_5$ | $Q_4$ | Smp. 116-117,5°C |
| 78 | $-C_2H_5$ | CP. | $Q_2$ | Smp. 84-86°C |
| 79 | $-CH_2OC_2H_5$ | $-CH_3$ | $Q_2$ | |
| 80 | 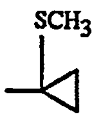 | $-CH_3$ | $Q_2$ | |
| 81 | $-C_4H_9$-n | CP. | $Q_1$ | |
| 82 | 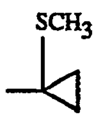 | $-CH_2OC_2H_5$ | $Q_1$ | |
| 83 | $-CH_3$ | $-CH_2OCHF_2$ | $Q_4$ | |
| 84 | $-CH_2OCHF_2$ | $-CH_2OCH_3$ | $Q_4$ | |
| 85 | $-CH_2OCH_2$ 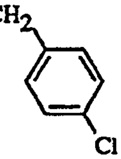 | $-CH_3$ | $Q_4$ | |
| 86 | CP. | CP. | $Q_1$ | Smp. 86-88°C |
| 87 | 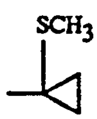 | $-CH_3$ | $Q_4$ | |
| 88 | $-CH_3$ | $-CH_2OCH_2CF_3$ | $Q_4$ | |
| 89 | $-CH_2OCH_2CF_3$ | CP. | $Q_4$ | |
| 90 | $-C_2H_5$ | CP. | $Q_4$ | Smp. 81-83°C |

| Verb. Nr. | $R_1$ | $R_2$ | $R_3$ | Physikal. Konstante |
|---|---|---|---|---|
| 91 | 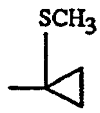 | $-CH_2OC_2H_5$ | $Q_4$ | |
| 92 | $-C_4H_9$-n | CP. | $Q_4$ | |
| 93 | $-CH_2OC_2H_5$ | $-CH_3$ | $Q_3$ | $n_D^{25}$ 1,5723 |
| 94 | $-C_2H_5$ | CP. | $Q_1$ | Smp. 73-75°C |
| 95 | $-CH_2OCH_3$ | CP. | $Q_1$ | Smp. 81-83°C |
| 96 | $-CH_2OC_2H_5$ | $-CH_3$ | $Q_4$ | |
| 97 | $-C_2H_5$ | $-CH_2OCH_3$ | $Q_1$ | |
| 98 | $-CH_2OCHF_2$ | $-CH_3$ | $Q_1$ | |
| 99 | $-CH_2OCH_2CF_3$ | $-CH_2OCH_3$ | $Q_4$ | |
| 100 | 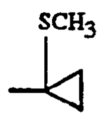 | $-C_2H_5$ | $Q_4$ | |
| 101 | $-CH_2OCHF_2$ | $-C_2H_5$ | $Q_4$ | |
| 102 | $-CH_2OCH(CH_3)-C_2H_5$ | $-CH_3$ | $Q_4$ | |
| 103 | $-CH_2OCH_2CH_2OCH_3$ | $-CH_3$ | $Q_1$ | |
| 104 | $-CH_2OCH_3$ | $-C_2H_5$ | $Q_4$ | Smp. 111-112°C |
| 105 | $-CH_2OCHF_2$ | $-CH_3$ | $Q_4$ | |
| 106 | $-CH_2OCH_2CF_3$ | $-C_2H_5$ | $Q_4$ | |
| 107 | CP. | $-C_3H_7$-iso | $Q_4$ | Smp. 108-111°C |
| 108 | $-CH_2OCHF_2$ | $-C_3H_7$-iso | $Q_1$ | |
| 109 | $-CH_2OC_2H_5$ | $-C_2H_5$ | $Q_1$ | |

| Verb. Nr. | $R_1$ | $R_2$ | $R_3$ | Physikal. Konstante |
|---|---|---|---|---|
| 110 | $-CH_2OCHF_2$ | $-CH_3$ | $Q_2$ | Oel |
| 111 | $-CH_2OC_2H_5$ | $-C_2H_5$ | $Q_4$ | Smp. 126-129°C |
| 112 | 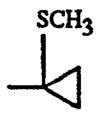 | $-C_3H_7$-n | $Q_4$ | |
| 113 | $-CH_2OCH_3$ | $-C_3H_7$-iso | $Q_4$ | |
| 114 | $-CH_2OCHF_2$ | $-C_3H_7$-iso | $Q_4$ | |
| 115 | $-CH_2OCH_2CH_2OCH_3$ | $-C_2H_5$ | $Q_4$ | |
| 116 | $-CH_2OC_2H_5$ | $-C_3H_7$-iso | $Q_4$ | |
| 117 | $-CH_2OCH_2CF_3$ | $-CH_3$ | $Q_1$ | |

| Verb. Nr. | $R_1$ | $R_2$ | $R_3$ | Physikal. Konstante |
|---|---|---|---|---|
| 118 | $-CH_2OC_2H_5$ | $-C_2H_5$ | $Q_3$ | Smp. 96-97°C |
| 119 | $-CH_2OCH_3$ | $-C_2H_5$ | $Q_3$ | Smp. 78-83°C |
| 120 | CP. | $-C_2H_5$ | $Q_3$ | Smp. 106-109°C |
| 121 | $-CH_2OCH_3$ | CP. | $Q_3$ | Smp. 104-106°C |
| 122 | $-CH_2OCH_3$ | $-C_2H_5$ | $Q_2$ | Smp. 81-83°C |
| 123 | $-CH_2OCH_3$ | CP. | $Q_2$ | Smp. 105-106°C |
| 124 | $-CH_2OCH_3$ | $-C_2H_5$ | $Q_1$ | Smp. 104-105°C |
| 125 | $-C_3H_7$iso | CP. | $Q_1$ | Smp. 89-92°C |
| 126 | $-C_3H_7$iso | CP. | $Q_3$ | Smp. 129-131°C |
| 127 | $-C_3H_7$iso | CP. | $Q_2$ | Smp. 109-110°C |
| 128 | $-C_3H_7$n | CP. | $Q_1$ | Smp. 86-87°C |
| 129 | $-C_3H_7$n | CP. | $Q_3$ | Smp. 74-76°C |
| 130 | $-C_3H_7$n | CP. | $Q_2$ | Smp. 81-83°C |
| 131 | $-CH_2OCHF_2$ | $CH_3$ | $Q_3$ | |
| 132 | $-CH_2OCHF_2$ | $C_2H_5$ | $Q_1$ | |
| 133 | $-CH_2OCHF_2$ | $C_2H_5$ | $Q_2$ | |
| 134 | $-CH_2OCHF_2$ | $C_2H_5$ | $Q_3$ | |
| 135 | $-CH_2OCHF_2$ | CP. | $Q_1$ | |
| 136 | $-CH_2OCHF_2$ | CP. | $Q_2$ | |
| 137 | $-CH_2OCHF_2$ | CP. | $Q_3$ | |

| Verb. Nr. | $R_1$ | $R_2$ | $R_3$ | Physikal. Konstante |
|---|---|---|---|---|
| 138 | CP. | CP. | $Q_2$ | Smp. 103-105°C |
| 139 | CP. | CP. | $Q_3$ | Smp. 123-126°C |
| 140 | CP. | $-C_3H_7$-n | $Q_1$ | Smp. 106-108°C |
| 141 | CP. | $-C_3H_7$iso | $Q_2$ | Smp. 108-110°C |
| 142 | CP. | $-C_3H_7$iso | $Q_1$ | Smp. 97-100°C |
| 143 | $-CH_2OCH_3$ | $-C_3H_7$iso | $Q_3$ | Smp. 89-91°C |
| 144 | CP. | $-C_3H_7$-n | $Q_2$ | Smp. 111-114°C |
| 145 | CP. | $-C_3H_7$iso | $Q_3$ | Smp. 101-103°C |
| 146 | $-CH_2OC_2H_5$ | CP. | $Q_1$ | Smp. 116-118°C |
| 147 | $-CH_2OCH_3$ | $-C_3H_7$-n | $Q_3$ | Smp. 98-101°C |
| 148 | $-CH_2OC_2H_5$ | CP. | $Q_3$ | Smp. 123-125°C |
| 149 | $-CH_2OCH_3$ | $-C_3H_7$iso | $Q_1$ | Smp. 87-90°C |
| 150 | $-CH_2OCH_3$ | $-C_3H_7$-n | $Q_1$ | Smp. 91-94°C |
| 151 | CP. | $-C_3H_7$-n | $Q_3$ | Smp. 103-106°C |
| 152 | $-CH_2OCH_3$ | $-C_3H_7$-n | $Q_2$ | Smp. 85-87°C |
| 153 | $-CH_2OCH_3$ | $-C_3H_7$iso | $Q_2$ | Smp. 93-95°C |
| 154 | CP. | $-C_3H_7$-n | $Q_4$ | Smp. 114-120°C |
| 155 | $-CH_2OCH_3$ | $-C_3H_7$-n | $Q_4$ | Smp. 124-126°C |
| 156 | $-CH_2OC_2H_5$ | CP. | $Q_2$ | Smp. 109-111°C |

Tabelle 2: Zwischenprodukte der Formel V

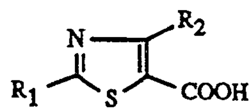

CP.= Cyclopropyl

| Verb. Nr. | $R_1$ | $R_2$ | Physikal. Konstante |
|---|---|---|---|
| 2.1 | CP. | $CH_3$ | Smp. 198-200°C |
| 2.2 | CP. | $C_2H_5$ | Smp. 96-97°C |
| 2.3 | CP. | $C_3H_7$-n | Smp. 104-106°C |
| 2.4 | CP. | $C_3H_7$-iso | Smp. 84-87°C |
| 2.5 | $CH_3$ | CP. | Smp. 190-191°C |
| 2.6 | $C_2H_5$ | CP. | Smp. 158-159°C |
| 2.7 | $C_3H_7$-iso | CP. | Smp. 122-123°C |
| 2.8 | $C_3H_7$-n | CP. | Smp. 161-162°C |
| 2.9 | $-CH_2OCH_3$ | $CH_3$ | Smp. 168-169,5°C |
| 2.10 | $-CH_2OCH_3$ | $C_2H_5$ | Smp. 146-147°C |
| 2.11 | $-CH_2OCH_3$ | CP. | Smp. 159-161°C |
| 2.12 | $-CH_2OC_2H_5$ | $CH_3$ | Smp. 185-186°C |
| 2.13 | $-CH_2OC_2H_5$ | $C_2H_5$ | Smp. 114-115°C |
| 2.14 | $-CH_2OC_2H_5$ | CP. | Smp. 132-134°C |
| 2.15 | CP. | CP. | Smp. 102-104°C |
| 2.16 | $-CH_2OCHF_2$ | $CH_3$ | |
| 2.17 | $-CH_2OCHF_2$ | $C_2H_5$ | |
| 2.18 | $-CH_2OCHF_2$ | CP. | |
| 2.19 | $-CH_2OCH_3$ | $C_3H_7$-n | Smp. 137-139°C |
| 2.20 | $-CH_2OCH_3$ | $C_3H_7$-iso | Smp. 148-151°C |

In Gegenwart eines sauren katalysators wie HCl lassen sich aus den Säuren der Tabelle 2 durch Veresterung mit Methanol, Ethanol oder einem der isomeren Alkohole Propanol, Pentanol oder Hexanol leicht die entsprechenden Carbonsäure-$C_1$-$C_8$-Alkylester gewinnen.

2.Formulierungsbeispiele für Wirkstoffe der Formel I (% = Gewichtsprozent)

| 2.1. Spritzpulver | a) | b) | c) |
|---|---|---|---|
| Wirkstoff aus der Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2.2. Emulsions-Konzentrat | |
|---|---|
| Wirkstoff aus der Tabelle 1 | 10 % |
| Octylphenolpolyethylenglykolether (4-5 Mol Ethylenoxid) | 3 % |
| Ca-Dodecylbenzolsulfonat | 3 % |
| Cyclohexanon | 34 % |
| Xylolgemisch | 50 % |

Aus diesem Konzentrat können durch Verdünnen mit Wasser Emulsionen jeder gewünschten Konzentration hergestellt werden.

| 2.3. Stäubemittel | a) | b) |
|---|---|---|
| Wirkstoff aus der Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt auf einer geeigneten Mühle vermahlen wird.

| 2.4. Extruder Granulat | |
|---|---|
| Wirkstoff aus der Tabelle 1 | 10 % |
| N-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

2.5. Umhüllungs-Granulat

| | |
|---|---|
| Wirkstoff aus der Tabelle 1 | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

2.6. Suspensions-Konzentrat

| | |
|---|---|
| Wirkstoff aus der Tabelle 1 | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig ver- mischt. Man erhält so ein Suspensions-Konzentrat, aus welchem durch Verdünnen mit Wasser Suspensionen jeder gewünschten Konzentration hergestellt werden können.

3. Biologische Beispiele

I. Wirkung gegen Phytophthora infestans auf Tomaten

a) Kurative Wirkung

Tomatenpflanzen der Sorte "Roter Gnom" werden nach dreiwöchiger Anzucht mit einer Zoosporensuspension des Pilzes besprüht und in einer Kabine bei 18 bis 20° und gesättigter Luftfeuchtigkeit inkubiert. Unterbruch der Befeuchtung nach 24 Stunden. Nach dem Abtrocknen der Pflanzen werden diese mit einer Brühe besprüht, die die als Spritzpulver formulierte Wirksubstanz in einer Konzentration von 600, 200 oder 60 ppm enthält. Nach dem Antrocknen des Spritzbelages werden die Pflanzen wieder in der Feuchtkabine während 4 Tagen aufgestellt. Anzahl und Grösse der nach dieser Zeit aufgetretenen typischen Blattflecken sind der Bewertungsmassstab für die Wirksamkeit der geprüften Substanzen.

b) Präventiv-Systemische Wirkung

Die als Spritzpulver formulierte Wirksubstanz wird in einer Konzentration von 60 ppm (bezogen auf das Bodenvolumen) auf die Bodenoberfläche von drei Wochen alten eingetopften Tomatenpflanzen der Sorte "Roter Gnom" gegeben. Nach dreitägiger Wartezeit wird die Blattunterseite der Pflanzen mit einer Zoosporensuspension von Phytophthora infestans besprüht. Sie wurden dann 5 Tage in einer Sprühkabine bei 18 bis 20°C und gesättigter Luftfeuchtigkeit gehalten. Nach dieser Zeit bilden sich typische Blattflecken, deren Anzahl und Grösse zur Bewertung der Wirksamkeit der geprüften Substanzen dienen.

Aus der Tabelle 1 erzielten folgende Verbindungen eine Hemmung des Krankheitsbefalls auf unter 10% : Nr. 2, 3, 5, 8, 9, 12, 18, 24, 32, 34, 35, 39, 48, 54, 55, 63, 68, 77, 78, 90, 93, 95, 98, 104, 107, 110, 111, 113, 116, 118 bis 130 und 138 bis 156.

II. Wirkung gegen Plasmopara viticola (Bert. et Curt.) (Berl. et DeToni) auf Reben

a) Residual-präventive Wirkung

Im Gewächshaus werden Rebenstecklinge der Sorte "Chasselas" herangezogen. Im 10-Blatt-Stadium wurden 3 Pflanzen mit einer aus der als Spritzpulver formulierten Wirksubstanz hergestellten Brühe (200 ppm Wirkstoff) besprüht. Nach dem Antrocknen des Spritzbelages werden die Pflanzen auf der Blattunterseite mit der Sporensuspension des Pilzes gleichmässig infiziert. Die Pflanzen werden anschliessend während 8 Tagen in einer Feuchtkammer gehalten. Nach dieser Zeit zeigen sich deutliche Krankheitssymptome an den Kontrollpflanzen. Anzahl und Grösse der Infektionsstellen an den behandelten Pflanzen dienen als Bewertungsmassstab für die Wirksamkeit der geprüften Substanzen.

b) Kurative Wirkung

Rebenstecklinge der Sorte "Chasselas" werden im Gewächshaus herangezogen und im 10-Blatt-Stadium mit einer Sporensuspension von Plasmopara viticola an der Blattunterseite infiziert. Nach 24 Stunden Aufenthalt in der Feuchtkabine werden die Pflanzen mit einer Wirkstoffbrühe besprüht, die aus einem Spritzpulver des Wirkstoffs hergestellt worden war (500 pm Wirkstoff). Anschliessend werden die Pflanzen 7 Tage weiterhin in der Feuchtkabine gehalten. Nach dieser Zeit zeigen sich die Krankheitssymptome an den Kontrollpflanzen. Anzahl und Grösse der Infektionsstellen an den behandelten Pflanzen dienen als Bewertungsmassstab für die Wirksamkeit der geprüften Substanzen.

Auch hier erzielten sämtliche im Beispiel I genannten Verbindungen eine Hemmung des Krankheitsbefalls auf unter 10%.

III. Wirkung gegen Pythium debaryanum auf Zuckerrüben (Beta vulgaris)

a) Wirkung nach Bodenapplikation

Der Pilz wird auf sterilen Haferkörnern kultiviert und einer Erde-Sand-Mischung beigegeben. Die so infizierte Erde wird in Blumentöpfe abgefüllt und mit Zuckerrübensamen besät. Gleich nach der Aussaat werden die als Spritzpulver formulierten Versuchspräparate als wässerige Suspension über die Erde gegossen (20 ppm Wirkstoff bezogen auf das Erdvolumen). Die Töpfe werden darauf während 2-3 Wochen im Gewächshaus bei 20-24°C aufgestellt. Die Erde wird ständig durch leichtes Besprühen mit Wasser gleichmässig feucht gehalten. Bei der Auswertung der Tests wird der Auflauf der Zuckerrübenpflanzen sowie der Anteil gesunder und kranker Pflanzen bestimmt.

b) Wirkung nach Beizapplikation

Der Pilz wird auf sterilen Haferkörnern kultiviert und einer Erde-Sand-Mischung beigegeben. Die so infizierte Erde wird in Blumentöpfe abgefüllt und mit Zuckerrübensamen besät, die mit den als Beizpulver formulierten Versuchspräparaten gebeizt worden waren (1000 ppm Wirkstoff bezogen auf dasSamengewicht). Die besäten Töpfe wurden während 2-3 Wochen im Gewächshaus bei 20-24°C aufgestellt. Die Erde wurde dabei durch leichtes Besprühen mit Wasser gleichmässig feucht gehalten. Bei der Auswertung wird der Auflauf der Zuckerrübenpflanzen sowie der Anteil gesunder und kranker Pflanzen bestimmt.

Mit Verbindungen aus der Tabelle 1 wurde eine Auflaufrate von über 80% erzielt. Die entsprechenden Kontrollpflanzen hatten eine Auflaufrate von weniger als 30% und ein ungesundes Aussehen.

## Ansprüche

1. Thiazolyl-5-carbonamid-Derivate der Formel I und ihre Säureadditionssalze und Metallsalzkomplexe

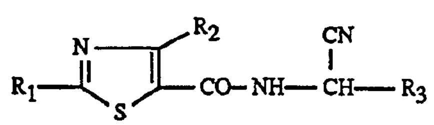

(I)

worin

$R_3$ 2-Furanyl, 2-Thienyl, 3-Furanyl oder 3-Thienyl bedeutet ; und worin

$R_1$ und $R_2$ unabhängig voneinander folgende Bedeutung haben :

a) $C_3$-$C_6$Cycloalkyl, das unsubstituiert oder durch Methyl oder Methylthio substituiert ist,

b) die Gruppe $-CH_2$-X-$R_4$, und

c) einer der beiden Substituenten $R_1$ oder $R_2$ auch Wasserstoff oder $C_1$-$C_4$Alkyl, wobei ferner X Sauerstoff oder Schwefel darstellt,

$R_4$ $C_1$-$C_4$Alkyl bedeutet, das unsubstituiert oder durch Halogen oder im Falle $C_2$-$C_4$Alkyl auch durch $C_1$-$C_3$Alkoxy substituiert sein kann, oder worin $R_4$ $C_3$-$C_4$Alkenyl, $C_3$-$C_4$Alkinyl oder eine Phenylgruppe oder Benzylgruppe darstellt, deren aromatischer Ring unsubstituiert ist oder durch Halogen, $C_1$-$C_2$Alkyl, $C_1$-$C_2$Alkoxy, $CF_3$ oder $NO_2$ substituiert sein kann.

2. Verbindungen der Formel I gemäss Anspruch 1, worin einer der beiden Substituenten $R_1$ und $R_2$ $C_3$-$C_6$Cycloalkyl bedeutet, das unsubstituiert oder durch Methyl oder Methylthio substituiert ist, und der andere Substituent
   d) Cyclobutyl, Cyclopropyl, Methylcyclopropyl oder Methylthiocyclopropyl,
   e) die Gruppe $—CH_2$-X-$R_4$ oder
   f) Wasserstoff oder $C_1$-$C_4$Alkyl darstellt, während
   $R_3$, X und $R_4$ die vorgenannte Bedeutung haben.

3. Verbindungen der Formel I gemäss Anspruch 2, worin einer der beiden Substituenten $R_1$ und $R_2$ $C_3$-$C_6$Cycloalkyl und der andere Cyclobutyl oder Cyclopropyl bedeutet, während $R_3$ die genannte Bedeutung hat.

4. Verbindungen der Formel I gemäss Anspruch 3, worin $R_1$ und $R_2$ Cyclopropyl bedeuten.

5. Verbindungen der Formel I gemäss Anspruch 2, worin
   $R_1$ Wasserstoff, $C_1$-$C_4$Alkyl oder die Gruppe $—CH_2$-X-$R_4$ darstellt,
   X Sauerstoff oder Schwefel bedeutet, und
   $R_4$ $C_1$-$C_4$Alkyl bedeutet, das unsubstituiert oder durch Fluor substituiert ist, oder im Falle $C_2$-$C_4$Alkyl auch durch $C_1$-$C_3$Alkoxy substituiert sein kann ; oder worin $R_4$ Allyl, Propargyl, Phenyl oder Benzyl darstellt, wobei die aromatischen Ringe der letztgenannten Reste unsubstituiert oder durch einen oder zwei der Substituenten Fluor, Chlor, Methyl, Ethyl, Methoxy oder $CF_3$ substituiert sein können, während
   $R_2$ $C_3$-$C_6$Cycloalkyl bedeutet, das unsubstituiert oder durch Methyl oder Methylthio substituiert ist, während $R_3$ die genanne Bedeutung hat.

6. Verbindungen gemäss Anspruch 5, worin X Sauerstoff bedeutet.

7. Verbindungen gemäss Anspruch 6, worin
   $R_1$ Wasserstoff, $C_1$-$C_4$Alkyl oder $—CH_2$-O-$R_4$ bedeutet,
   $R_2$ Cyclobutyl, Cyclopropyl, Methylcyclopropyl oder Methylthiocyclopropyl darstellt, und
   $R_4$ $C_1$-$C_4$Alkyl bedeutet, das unsubstituiert oder fluorsubstituiert ist oder, im Falle $C_2$-$C_4$Alkyl, auch durch Methoxy oder Ethoxy substituiert sein kann ; oder worin ferner
   $R_4$Allyl, Propargyl, Phenyl, Chlorphenyl, Fluorphenyl, Benzyl, Chlorbenzyl oder Fluorbenzyl ist, während
   $R_3$ die genannte Bedeutung hat.

8. Verbindungen gemäss Anspruch 7, worin
   $R_2$ Cyclopropyl bedeutet, und
   $R_4$ Methyl, Ethyl, Fluormethyl, Difluormethyl, Trifluorethyl, Allyl, Propargyl, Phenyl oder Benzyl ist.

9. Verbindungen gemäss Anspruch 8, worin $R_1$ Wasserstoff, $C_1$-$C_3$Alkyl, Methoxymethyl oder Ethoxymethyl bedeutet.

10. Verbindungen der Formel I gemäss Anspruch 1, worin einer der beiden Substituenten $R_1$ und $R_2$ die Gruppe $—CH_2$-X-$R_4$ und der andere Substituent Wasserstoff, $C_1$-$C_4$Alkyl oder $—CH_2$-X-$R_4$ bedeutet,
    X Sauerstoff oder Schwefel darstellt,
    $R_3$ die genannte Bedeutung hat, und
    $R_4$ $C_1$-$C_4$Alkyl bedeutet, das unsubstituiert oder durch Fluor substituiert ist, oder im Falle $C_2$-$C_4$Alkyl auch durch $C_1$-$C_3$Alkoxy substituiert sein kann ; oder worin $R_4$ Allyl, Propargyl, Phenyl oder Benzyl darstellt, wobei die aromatischen Ringe der letztgenannten Reste unsubstituiert oder durch einen oder zwei der Substituenten Fluor, Chlor, Methyl, Ethyl, Methoxy oder $CF_3$ substituiert sein können.

11. Verbindungen gemäss Anspruch 10, worin
    $R_1$ die Gruppe $—CH_2$-O-$R_4$ bedeutet,
    $R_2$ Wasserstoff, $C_1$-$C_4$Alkyl oder $—CH_2$-O-$R_4$ ist,
    $R_3$ die genannte Bedeutung hat, und
    $R_4$ $C_1$-$C_4$Alkyl bedeutet, das unsubstituiert oder durch Fluor, Methoxy oder Ethoxy substituiert ist, oder worin $R_4$ Allyl, Propargyl, Phenyl, Chlorphenyl, Fluorphenyl, Benzyl, Chlorbenzyl oder Fluorbenzyl bedeutet.

12. Verbindungen gemäss Anspruch 11, worin
$R_2$ Wasserstoff oder $C_1$-$C_3$Alkyl bedeutet.

13. Verbindungen der Formel I gemäss Anspruch 1, worin entweder
a) $R_1$ $CH_3$, $C_2H_5$, n-Propyl, iso$C_3H_7$, $CH_2$-O-$CH_3$, $CH_2$-O-$C_2H_5$, $CH_2$-O-$CHF_2$ oder Cyclopropyl, und
$R_2$ Cyclopropyl bedeuten, oder worin
b) $R_1$ $CH_2$-O-$CH_3$, $CH_2$-O-$C_2H_5$, $CH_2$-O-$CHF_2$ oder Cyclopropyl und
$R_2$ $C_1$-$C_3$-Alkyl darstellen, und
$R_3$ die genannte Bedeutung hat.

14. Verbindungen der Formel I gemäss Anspruch 13, worin $R_1$ Methyl, Ethyl, n-Propyl oder Isopropyl und $R_2$ Cyclopropyl darstellt.

15. Verbindungen der Formel I gemäss Anspruch 13, worin
$R_1$ $CH_2$-O-$CH_3$, $CH_2$-O-$C_2H_5$ oder Cyclopropyl und
$R_2$ Methyl, Ethyl, n-Propyl, Isopropyl oder Cyclopropyl bedeuten.

16. Verfahren zur Herstellung von Thiazolyl-5-carbonamid-Derivaten der Formel I gemäss Anspruch 1, gekennzeichnet durch Reaktion der Thiazolyl-5-carbonsäure der Formel V

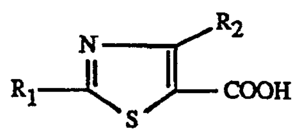

(V)

oder ihres Säurehalogenids, Esters oder Säureanhydrids mit einem Aminoacetonitril der Formel II

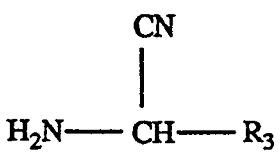

(II)

in Gegenwart oder Abwesenheit eines Kondensationsmittel bzw. eines Säureakzeptors, wobei die Substituenten $R_1$, $R_2$ und $R_3$ die für die Formel I gegebene Bedeutung haben.

17. Mikrobizides Mittel zur Bekämpfung von Pflanzenkrankheiten enthaltend als Wirkstoff eine Verbindung der Formel I gemäss Anspruch 1 zusammen mit einem geeigneten Trägermaterial.

18. Mittel gemäss Anspruch 17 enthaltend als Wirkstoff eine Verbindung gemäss einem der Ansprüche 2 bis 15.

19. Verwendung einer Verbindung der Formel I zur Bekämpfung von Mikroorganismen und zur Verhütung von Mirkoorganismenbefall.

20. Verfahren zur Bekämpfung von Pflanzenkrankheiten bzw. zur Verhütung von Krankheitsbefall, gekennzeichnet durch Applikation einer Verbindung der Formel I an Pflanzen, an den Ort ihres Wachstums, an Pflanzenteile oder auf Pflanzen- Vermehrungsgut.

21. Verfahren gemäss Anspruch 20, wobei das Vermehrungsgut das Saatgut ist.

22. Verbindungen der Formel V

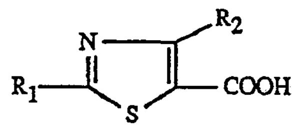

(V)

und ihre $C_1$-$C_6$ Carbonsäureester, wobei $R_1$ und $R_2$ die für Formel I gemäss Anspruch 1 gegebene Bedeutung haben.

23. Verbindungen der Formel V und ihre $C_1$-$C_6$-Carbonsäureester gemäss Anspruch 22, worin $R_1$ und $R_2$ die für Formel I gemäss Anspruch 13 gegebene Bedeutung haben.

**Ansprüche für folgenden Vertragsstaat : ES**

1. Verfahren zur Herstellung von Thiazolyl-5-carbonamid-Derivaten der Formel I und ihre Säureadditionssalze und Metallsalzkomplexe

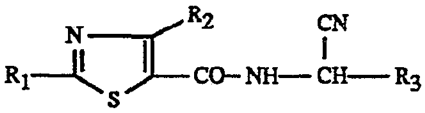

(I)

worin
$R_3$ 2-Furanyl, 2-Thienyl, 3-Furanyl oder 3-Thienyl bedeutet ; und worin
$R_1$ und $R_2$ unabhängig voneinander folgende Bedeutung haben :
a) $C_3$-$C_6$Cycloalkyl, das unsubstituiert oder durch Methyl oder Methylthio substituiert ist,
b) die Gruppe —$CH_2$-X-$R_4$, und
c) einer der beiden Substituenten $R_1$ oder $R_2$ auch Wasserstoff oder $C_1$-$C_4$Alkyl, wobei ferner X Sauerstoff oder Schwefel darstellt,
$R_4$ $C_1$-$C_4$Alkyl bedeutet, das unsubstituiert oder durch Halogen oder im Falle $C_2$-$C_4$Alkyl auch durch $C_1$-$C_3$Alkoxy substituiert sein kann, oder worin $R_4$ $C_3$-$C_4$Alkenyl, $C_3$-$C_4$Alkinyl oder eine Phenylgruppe oder Benzylgruppe darstellt, deren aromatischer Ring unsubstituiert ist oder durch Halogen, $C_1$-$C_2$Alkyl, $C_1$-$C_2$Alkoxy, $CF_3$ oder $NO_2$ substituiert sein kann, gekennzeichnet durch Reaktion der Thiazolyl-5-carbonsäure der Formel V

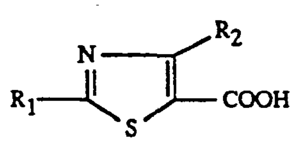

(V)

oder ihres Säurehalogenids, Esters oder Säureanhydrids mit einem Aminoacetonitril der Formel II

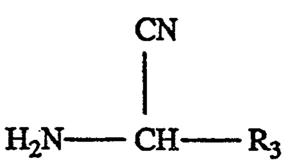

(II)

in Gegenwart oder Abwesenheit eines Kondensationsmittel bzw. eines Säureakzeptors, wobei die Substituenten $R_1$, $R_2$ und $R_3$ die für die Formel I gegebene Bedeutung haben, und, sofern gewünscht, Ueberführung der so gewonnenen Endprodukte der Formel I in ihre Säureadditionssalze bzw. Metallkomplexe.

2. Verfahren gemäss Anspruch 1, dadurch gekennzeichnet, dass Verbindungen der Formel I hergestellt werden, worin einer der beiden Substituenten $R_1$ und $R_2$ $C_3$-$C_6$Cycloalkyl bedeutet, das unsubstituiert oder durch Methyl oder Methylthio substituiert ist, und der andere Substituent
d) Cyclobutyl, Cyclopropyl, Methylcyclopropyl oder Methylthiocyclopropyl,

e) die Gruppe —$CH_2$-X-$R_4$ oder
f) Wasserstoff oder $C_1$-$C_4$Alkyl darstellt, während
$R_3$, X und $R_4$ die vorgenannte Bedeutung haben.

3. Verfahren gemäss Anspruch 1, dadurch gekennzeichnet, dass Verbindungen der Formel I hergestellt werden, worin einer der beiden Substituenten $R_1$ und $R_2$ $C_3$-$C_6$Cycloalkyl und der andere Cyclobutyl oder Cyclopropyl bedeutet, während $R_3$ die genannte Bedeutung hat.

4. Verfahren gemäss Anspruch 1, dadurch gekennzeichnet, dass Verbindungen der Formel I hergestellt werden, worin $R_1$ und $R_2$ Cyclopropyl bedeuten.

5. Verfahren gemäss Anspruch 1, dadurch gekennzeichnet, dass Verbindungen der Formel I hergestellt werden, worin
$R_1$ Wasserstoff, $C_1$-$C_4$Alkyl oder die Gruppe —$CH_2$-X-$R_4$ darstellt,
X Sauerstoff oder Schwefel bedeutet, und
$R_4$ $C_1$-$C_4$Alkyl bedeutet, das unsubstituiert oder durch Fluor substituiert ist, oder im Falle $C_2$-$C_4$Alkyl auch durch $C_1$-$C_3$Alkoxy substituiert sein kann ; oder worin $R_4$ Allyl, Propargyl, Phenyl oder Benzyl darstellt, wobei die aromatischen Ringe der letztgenannten Reste unsubstituiert oder durch einen oder zwei der Substituenten Fluor, Chlor, Methyl, Ethyl, Methoxy oder $CF_3$ substituiert sein können, während
$R_2$ $C_3$-$C_6$Cycloalkyl bedeutet, das unsubstituiert oder durch Methyl oder Methylthio substituiert ist, während $R_3$ die genanne Bedeutung hat.

6. Verfahren gemäss Anspruch 1, dadurch gekennzeichnet, dass Verbindungen gemäss Anspruch 5 hergestellt werden, worin X Sauerstoff bedeutet.

7. Verfahren gemäss Anspruch 1, dadurch gekennzeichnet, dass Verbindungen gemäss Anspruch 6 hergestellt werden, worin
$R_1$ Wasserstoff, $C_1$-$C_4$Alkyl oder —$CH_2$-O-$R_4$ bedeutet,
$R_2$ Cyclobutyl, Cyclopropyl, Methylcyclopropyl oder Methylthiocyclopropyl darstellt, und
$R_4$ $C_1$-$C_4$Alkyl bedeutet, das unsubstituiert oder fluorsubstituiert ist oder, im Falle $C_2$-$C_4$Alkyl, auch durch Methoxy oder Ethoxy substituiert sein kann ; oder worin ferner
$R_4$ Allyl, Propargyl, Phenyl, Chlorphenyl, Fluorphenyl, Benzyl, Chlorbenzyl oder Fluorbenzyl ist, während
$R_3$ die genannte Bedeutung hat.

8. Verfahren gemäss Anspruch 1, dadurch gekennzeichnet, dass Verbindungen gemäss Anspruch 7 hergestellt werden, worin
$R_2$ Cyclopropyl bedeutet, und
$R_4$ Methyl, Ethyl, Fluormethyl, Difluormethyl, Trifluorethyl, Allyl, Propargyl, Phenyl oder Benzyl ist.

9. Verfahren gemäss Anspruch 1, dadurch gekennzeichnet, dass Verbindungen gemäss Anspruch 8 hergestellt werden, worin $R_1$ Wasserstoff, $C_1$-$C_3$Alkyl, Methoxymethyl oder Ethoxymethyl bedeutet.

10. Verfahren gemäss Anspruch 1, dadurch gekennzeichnet, dass Verbindungen der Formel I hergestellt werden, worin einer der beiden Substituenten $R_1$ und $R_2$ die Gruppe —$CH_2$-X-$R_4$ und der andere Substituent Wasserstoff, $C_1$-$C_4$Alkyl oder —$CH_2$-X-$R_4$ bedeutet,
X Sauerstoff oder Schwefel darstellt,
$R_3$ die genannte Bedeutung hat, und
$R_4$ $C_1$-$C_4$Alkyl bedeutet, das unsubstituiert oder durch Fluor substituiert ist, oder im Falle $C_2$-$C_4$Alkyl auch durch $C_1$-$C_3$Alkoxy substituiert sein kann ; oder worin $R_4$ Allyl, Propargyl, Phenyl oder Benzyl darstellt, wobei die aromatischen Ringe der letztgenannten Reste unsubstituiert oder durch einen oder zwei der Substituenten Fluor, Chlor, Methyl, Ethyl, Methoxy oder $CF_3$ substituiert sein können.

11. Verfahren gemäss Anspruch 1, dadurch gekennzeichnet, dass Verbindungen gemäss Anspruch 10 hergestellt werden, worin
$R_1$ die Gruppe —$CH_2$-O-$R_4$ bedeutet,
$R_2$ Wasserstoff, $C_1$-$C_4$Alkyl oder —$CH_2$-O-$R_4$ ist,
$R_3$ die genannte Bedeutung hat, und

$R_4$ $C_1$-$C_4$Alkyl bedeutet, das unsubstituiert oder durch Fluor, Methoxy oder Ethoxy substituiert ist, oder worin $R_4$ Allyl, Propargyl, Phenyl, Chlorphenyl, Fluorphenyl, Benzyl, Chlorbenzyl oder Fluorbenzyl bedeutet.

12. Verfahren gemäss Anspruch 1, dadurch gekennzeichnet, dass Verbindungen gemäss Anspruch 11 hergestellt werden, worin
$R_2$ Wasserstoff oder $C_1$-$C_3$Alkyl bedeutet.

13. Verfahren gemäss Anspruch 1, dadurch gekennzeichnet, dass Verbindungen der Formel I hergestellt werden, worin entweder
a) $R_1$ $CH_3$, $C_2H_5$, n-Propyl, iso$C_3H_7$, $CH_2$-O-$CH_3$, $CH_2$-O-$C_2H_5$, $CH_2$-O-$CHF_2$ oder Cyclopropyl, und
$R_2$ Cyclopropyl bedeuten, oder worin
b) $R_1$ $CH_2$-O-$CH_3$, $CH_2$-O-$C_2H_5$, $CH_2$-O-$CHF_2$ oder Cyclopropyl und
$R_2$ $C_1$-$C_3$-Alkyl darstellen, und
$R_3$ die genannte Bedeutung hat.

14. Verfahren gemäss Anspruch 1, dadurch gekennzeichnet, dass Verbindungen der Formel I gemäss Anspruch 13 hergestellt werden, worin $R_1$ Methyl, Ethyl, n-Propyl oder Isopropyl und $R_2$ Cyclopropyl darstellt.

15. Verfahren gemäss Anspruch 1, dadurch gekennzeichnet, dass Verbindungen der Formel I gemäss Anspruch 13 hergestellt werden, worin
$R_1$ $CH_2$-O-$CH_3$, $CH_2$-O-$C_2H_5$ oder Cyclopropyl und
$R_2$ Methyl, Ethyl, n-Propyl, Isopropyl oder Cyclopropyl bedeuten.

16. Verfahren zur Bekämpfung von Pflanzenkrankheiten bzw. zur Verhütung von Krankheitsbefall, gekennzeichnet durch Applikation einer Verbindung der Formel I an Pflanzen, an den Ort ihres Wachstums, an Pflanzenteile oder auf Pflanzen-Vermehrungsgut.

17. Verfahren gemäss Anspruch 16, wobei das Vermehrungsgut das Saatgut ist.

18. Verfahren zur Herstellung von Verbindungen der Formel V

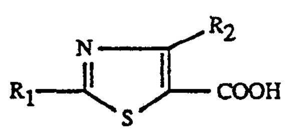

(V)

und ihren $C_1$-$C_8$ Carbonsäureestern, wobei $R_1$ und $R_2$ die für Formel I gemäss Anspruch 1 gegebene Bedeutung haben, gekennzeichnet durch Reaktion eines Thioamids der Formel VI mit einem α-Halogen-β-ketoester der Formel VII gemäss

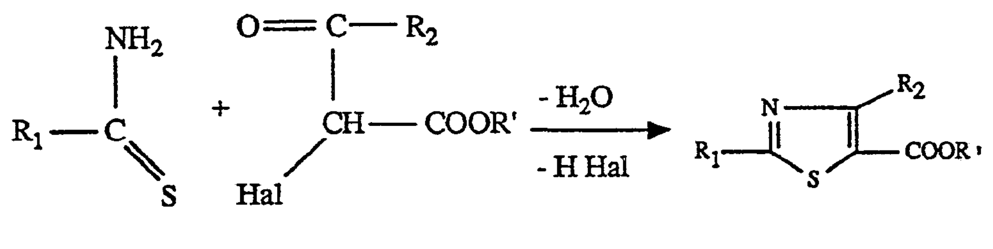


VI VII Va

wobei R' einen $C_1$-$C_8$ Kohlenwasserstoff bedeutet, und anschliessend, sofern gewünscht, Verseifen des Esters.